(19) Europäisches Patentamt
European Patent Office
Office européen des brevets

(11) Publication number: **0 169 603**
**B1**

(12) # EUROPEAN PATENT SPECIFICATION

(45) Date of publication of patent specification: **22.02.89**

(51) Int. Cl.$^4$: **C 07 D 205/04**

(21) Application number: **85201102.2**

(22) Date of filing: **05.07.85**

(54) **Preparation of N-substituted-3-cyano azetidine derivatives.**

(30) Priority: **26.07.84 GB 8419085**

(43) Date of publication of application:
**29.01.86 Bulletin 86/05**

(45) Publication of the grant of the patent:
**22.02.89 Bulletin 89/08**

(84) Designated Contracting States:
**AT BE CH DE FR GB IT LI LU NL SE**

(56) References cited:
**EP-A-0 029 265**
**EP-A-0 125 714**
**US-A-4 119 637**

**SYNTHESIS, INTERNATIONAL JOURNAL OF METHODS IN SYNTHETIC ORGANIC CHEMISTRY, no. 7, July 1975, pages 430-431; D. LANDINI et al.: "A convenient synthesis of optically active secondary alkyl halides under phase-transfer conditions"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Mason, Ronald Frank**
**"Kingswood"**
**Westwell Ashford-Kent (GB)**
Inventor: **Briner, Paul Howard**
**4, Meadowbank Cottages**
**Painters Forstal Faversham-Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 169 603 B1

**Description**

This invention relates to a process for the preparation of N-substituted-3-cyano azetidine derivatives, which are intermediates for the preparation of biologically active compounds.

It is known to prepare cyano compounds by nucleophilic displacement reaction between an alkali metal cyanide and a compound RX, wherein X is a suitable leaving group such as a halide or sulphonate. Such a reaction usually involves both aqueous and organic liquid phases, resulting in a relatively slow rate of reaction. An established technique to accelerate such biphase reactions is to use a phase-transfer catalyst, such as a quanternary ammonium salt. However, in some reactions the quaternary ammonium cation pairs with the leaving group anion to form a product which is very soluble in the organic phase, which necessitates the use of the quaternary ammonium phase transfer agent in considerably greater than purely catalytic amounts. This applies particularly with the displacement of tosyloxy or mesyloxy groups, since quaternary ammonium tosylates and mesylates are extremely soluble in organic solvents, and almost equimolar amounts of "catalyst" are required; see, for example, J. Chem. Soc., 954 (1962); Tetrahedron, *27*, 4057 (1971); Synthesis, 30 (1975). The mesyloxy group is often a commercially attractive choice of leaving group, but the need for such large proportions of phase transfer agent clearly imposes a serious economic handicap, and it would therefore be a commercially useful technical advance if this handicap could be overcome. The Applicants have now discovered that a selected group of phase transfer agents are effective at merely catalytic proportions in promoting the mesylate/cyanide displacement reaction.

Accordingly, the present invention provides a process for the preparation of N-substituted-3-cyano-azetidine derivatives of the formula I:

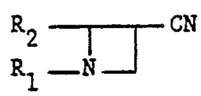

I

in which $R_1$ represents a benzyl group and $R_2$ represents a hydrogen atom or an alkyl, aryl or aralkyl group in which the alkyl moiety contains up to 8 carbon atoms, by reacting an N-substituted azetidine-3-ol of formula II:

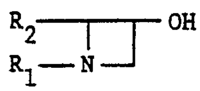

II

in a water-immiscible organic solvent with an alkylsulphonyl halide, followed by reaction of the resulting organic solution of the 3-alkylsulphonyl azetidine product with an aqueous solution of an alkali metal cyanide in the presence of, as a lipophilic phase transfer catalyst, a catalytic proportion of a tetraalkyl ammonium halide in which the alkyl groups contain a total of at least 20 carbon atoms.

Preferably $R_2$ represents a hydrogen atom; and the alkylsulphonyl halide is a chloride, in particular methylsulphonyl (mesyl) chloride.

The organic solvent used in the first, sulphonation, stage should be water-immiscible in order to enable the subsequent cyanation to be carried out in a two phase system, thereby facilitating the removal of excess cyanide ions and inorganic salts via the discarded aqueous phase. Most conventional water-immiscible organic solvents may be used, though it has been found that yields are less good with aliphatic chlorohydrocarbons. Thus suitable organic solvents include esters, such as ethyl acetate, and optionally halogenated aromatic hydrocarbons, such as toluene and chlorobenzene.

The alkali metal cyanide is, for reasons of commercial economics, usually sodium cyanide and, as already stated, is used in an aqueous solution and the resulting two phase reaction system is promoted by the presence of a phase transfer catalyst. Applicants have found that some widely used phase transfer catalysts are not effective in promoting the desired cyanation, and that this lack of efficacy appears to be found in those phase-transfer catalysts which are lipophobic, such as hexadecyltributylphosphonium bromide, tetrabutylammonium bromide, and crown ethers such as 18-crown-6. Accordingly, as specified above, the phase transfer catalyst should be lipophilic, i.e. organic soluble/water insoluble. Compounds suitable as phase transfer catalysts are tetraalkyl ammonium halides in which the alkyl groups contain a total of at least 20 carbon atoms, in particular such tetraalkyl ammonium halides which contain at least 1 methyl group and at least two alkyl groups containing at least 8 carbon atoms. Many of the commercially available quaternary ammonium salts are derived from natural products e.g. tallow, and therefore contain alkyl groups of varying chain length; the foregoing figures refer, in such cases, to the mean chain length of the different alkyl groups present.

The proportion of phase transfer catalyst may be within the levels normally used in the catalysis of such two phase systems, being significantly less than equimolar and normally not more than 20 mol%. Taking both practical and economic factors into account, preferred proportions are from 1 to 20, preferably from 2 to 10, mol%.

The first reaction step between the alcohol and the alkylsulphonyl halide is exothermic, and the 3-alkylsulphonyl azetidine formed is liable to partial decomposition at elevated temperatures. It is therefore

desirable to carry out this first step with cooling to prevent the reaction temperature exceeding room temperature (25°C), preferably cooling sufficiently to maintain a reaction temperature below 25°C but above 0°C, e.g. between 5°C and 15°C. The second reaction step is suitably carried out at elevated temperature, conveniently by refluxing the reaction mixture.

The starting azetidin-3-ol of formula II may be prepared by appropriate adaptations of known synthetic procedures. A preferred synthesis route to that product is described and claimed in Applicants' copending European Patent Application EP—A—161.722, namely by cyclizing a solution in triethylamine of an aminoalcohol of formula III:

$$R_1 - NH - \underset{\displaystyle R_2}{\underset{|}{CH}} - \underset{\displaystyle OH}{\underset{|}{CH}} - CH_2Hal \qquad III$$

(wherein $R_1$ and $R_2$ are as defined above and Hal denotes a halogen atom).

As mentioned above, the N-substituted-3-cyano-azetidine derivatives of formula I are useful intermediates. Thus, they may be converted by known procedures, for example, via the corresponding 3-carboxylic acid derivative, to azetidine-3-carboxylic and acid derivatives, which exhibit plant growth regulant properties, especially the property of rendering sterile the male parts of plants.

The invention therefore includes a process for the preparation of N-substituted azetidine-3-carboxylic acids and azetidine-3-carboxylic acid comprising hydrolysing the N-substituted-3-cyano-azetidine derivatives prepared by the process of the invention to the corresponding N-substituted azeitidine-3-carboxylic acid and optionally hydrogenating the N-substituted derivative to azetidine-3-carboxylic acid.

The invention is illustrated in the following Examples.

## Example 1

Preparation of N-benzyl-3-cyano azetidine

a) N-benzylazetidin-3-ol (4.75 kg), triethylamine (3.11 kg) and toluene (33 l) were charged into a reactor and stirred until the alcohol had dissolved. Methylsulphonyl chloride (3.51 kg) was added over 2 hours and the reaction mixture stirred for 2 hours with cooling to 7—12°C. The product was then submitted to phase separation, a water wash was applied, and the resulting toluene solution of N-benzyl-3-mesyloxy azetidine used directly for the subsequent cyanation step.

b) Further toluene (2 l) was added to the aqueous phase resulting from a), and charged to a reactor together with water (10 l), sodium cyanide (2.83 kg) and the phase transfer catalyst "Adogen" 464 (1.29 kg) dissolved in 2.8 l toluene). "Adogen" 464 is a proprietary trialkylmethyl ammonium chloride in which each alkyl group (except the methyl group) contains 8, 9 or 10 carbon atoms. "Adogen" is a registered Trade Mark. This mixture was refluxed (87°C) with vigorous agitation for 1 hour, then cooled to room temperature (23°C), and the phases separated. The organic phase was then washed with water and the solvent removed by evaporation. Distillation yielded 3.77 kg of the desired product, purity about 87%, a yield of 68% based on the starting azetidin-ol.

## Examples 2—10

Following procedures similar to those described in Example 1, but on a smaller scale, the suitability of alternative solvents and phase transfer agents was evaluated with the results set out in the Table below. Except where otherwise stated, the concentration of alcohol in the solvent was 1M, the mesylation temperature around 10°C, the amount of PTC used was 10 mol% and the cyanide was used at a 6 molar concentration in an amount to provide 2 equivalents. The phase transfer catalysts (PTC) used in Examples 8—10 are *not* lipophilic; these examples are therefore not illustrative of the invention but are included for the purposes of comparison with the yields obtained in the other Examples using the lipophilic PTCs according to this invention.

Table

| Example | Solvent | Phase Transfer Catalyst | Cyanation conditions | Yield |
|---|---|---|---|---|
| 2 | Ethyl Acetate | "Adogen" 464 | $^3/_4$hr reflux | 51 |
| 3 | Chlorobenzene | "Adogen" 464 | 30 min reflux | 62 |
| 4 | Toluene | $(C_7H_{15})_4NBr$ | 1hr reflux | 64 |
| 5 | Toluene | "Arquad" 2C-75/10 | 1hr reflux | 72 |
| 6 | Toluene | "Arquad" 2HT-75/10 | 1hr reflux | 68 |
| 7 | Toluene | $(C_{18}H_{37})_2(CH_3)_2NBr$ | 5hr reflux | 76 |
| 8 | Toluene | $(C_4H_9)_3C_{16}H_{33}PBr$ (5%) | 4hr 80°C | 33 |
| 9 | Toluene | 18-crown-6 (3%) | 18hr 80°C | 0 |
| 10 | Toluene | $(C_4H_9)_4NBr$ | 1hr reflux | 45 |

"Arquad" 2C-75/10 and 2HT-75/10 are dimethyl dialkyl ammonium chlorides in which the alkyl groups are derived, respectively, form coconut oil (average carbon chain length $C_{12}$ 50%; $C_{14}$ 20%) and hydrogenated tallow (average carbon chain length $C_{16}$ 31%, $C_{18}$ 64%. "Arquad" is a registered Trade Mark.

The results shown in the above Table demonstrate clearly that use of a water-soluble PTC (Examples 8—10) results in unacceptably low yields, whereas the lipophilic PTC of the present invention, especially using toluene as solvent, enables good yields to be achieved.

Example 11

Use of N-benzyl-3-cyanoazetidine to prepare azetidin-3-carboxylic acid

a) 1-Benzyl-3-cyanoazetidine (0.5 g) in saturated barium hydroxide solution (10 ml) was heated under reflux for 30 hours. The reaction mixture was cooled, saturated with gaseous carbon dioxide and filtered. The solvent was removed from the filtrate under reduced pressure to give 1-benzylazetidine-3-carboxylic acid in 80% yield.

b) 1-Benzylazetidine-3-carboxylic acid (0.5 g, prepared as in a)) in methanol (15 ml) was hydrogenated in the presence of a 5% palladium on carbon catalyst at room temperature. The catalyst was filtered off and the solvent removed from the filtrate under reduced pressure to give azetidin-3-carboxylic acid in 90% yield.

**Claims**

1. A process for the preparation of N-substituted-3-cyano-azetidine derivatives of the formula I:

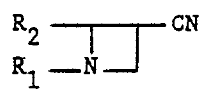

I

in which $R_1$ represents a benzyl group and $R_2$ represents a hydrogen atom or an alkyl, aryl or aralkyl group in which the alkyl moiety contains up to 8 carbon atoms, by reacting an N-substituted azetidin-3-ol derivative of formula II:

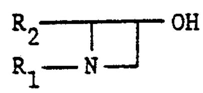

II

in a water-immiscible organic solvent with an alkylsulphonyl halide, followed by reaction of the resulting organic solution of the 3-alkylsulphonyl azetidine product with an aqueous solution of an alkali metal cyanide in the presence of, as a lipophilic phase transfer catalyst, a catalytic proportion of a tetraalkyl ammonium halide in which the alkyl groups contain a total of at least 20 carbon atoms.

2. Process as claimed in claim 1 wherein $R_2$ represents a hydrogen atom.

3. Process as claimed in claims 1 or 2 wherein the alkylsulphonyl halide is methylsulphonyl chloride.

4. Process as claimed in claims 1, 2 or 3 wherein the organic solvent is toluene or chlorobenzene.

5. Process as claimed in any one of the preceding claims wherein the tetraalkyl ammonium halide contains at least 2 alkyl groups containing at least 8 carbon atoms and at least one methyl group.

6. Process as claimed in any one of the preceding claims wherein the phase transfer catalyst is present in an amount of from 1 to 20 mol%, based on the starting alcohol.

7. Process as claimed in any one of the preceding claims wherein the reaction between the alcohol of formula II and the alkylsulphonyl halide is carried out with cooling to maintain the reaction temperature below 25°C, and the subsequent reaction with alkali metal cyanide is carried out at elevated temperature.

8. N-substituted-3-cyano azetidine derivatives of formula I, when prepared by a process as claimed in any one of claims 1—7.

9. A process for the preparation of N-substituted azetidine-3-carboxylic acids and azetidine-3-carboxylic acid comprising hydrolysing an N-substituted- 3-cyano azetidine of claim 8 to the corresponding N-substituted azetidine-3-carboxylic acid and optionally hydrogenating the N-substituted derivative to azetidine-3-carboxylic acid.

**Patentansprüche**

1. Verfahren zur Herstellung von N-substituierten 3-Cyano-azetidinderivaten der Formel I:

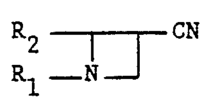

I

worin $R_1$ eine Benzylgruppe darstellt und $R_2$ ein Wasserstoffatom oder eine Alkyl-, Aryl- oder Aralkylgruppe

bedeutet, worin der Alkylrest bis zu 8 Kohlenstoffatome enthält, durch Umsetzung eines N-substituierten Azetidin-3-ols der Formel II:

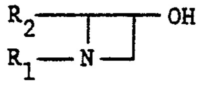

II

in einem mit Wasser nicht mischbaren organischen Lösungsmittel mit einem Alkylsulfonylhalogenid und anschließende Umsetzung der entstandenen organischen Lösung des 3-Alkylsulfonylazetidinproduktes mit einer wäßrigen Lösung eines Alkalimetallcyanids in Anwesenheit eines katalytischen Mengenanteiles eines Tetraalkylammoniumhalogenids, worin die Alkylgruppen insgesamt wenigstens 20 Kohlenstoffatome enthalten, als lipophilem Phasentransferkatalysator.

2. Verfahren nach Anspruch 1, worin $R_2$ ein Wasserstoffatom bedeutet.

3. Verfahren nach Anspruch 1 oder 2, worin das Alkylsulfonylhalogenid Methylsulfonylchlorid ist.

4. Verfahren nach Anspruch 1, 2 oder 3, worin das organische Lösungsmittel Toluol oder Chlorbenzol ist.

5. Verfahren nach einem der vorstehenden Ansprüche, worin das Tetraalkylammoniumhalogenid wenigstens zwei Alkylgruppen mit wenigstens 8 Kohlenstoffatomen und wenigstens eine Methylgruppe enthält.

6. Verfahren nach einem der vorstehenden Ansprüche, worin der Phasentransferkatalysator in einer Menge von 1 bis 20 Mol-%, bezogen auf den eingesetzten Alkohol, vorliegt.

7. Verfahren nach einem der vorstehenden Ansprüche, worin die Umsetzung zwischen dem Alkohol der Formel II und dem Alkylsulfonylhalogenid unter Kühlung zur Aufrechterhaltung der Reaktionstemperatur unter 25°C ausgeführt wird, und die anschließende Umsetzung mit Alkalimetallcyanid bei erhöhter Temperatur vorgenommen wird.

8. N-substituierte 3-Cyano-azetidinderivate der Formel I, wenn sie nach einem Verfahren, wie in einem der Ansprüche 1 bis 7 beansprucht, hergestellt sind.

9. Verfahren zur Herstellung von N-substituierten Azetidin-3-carbonsäuren und von Azetidin-3-carbonsäure, umfassend ein Hydrolysieren eines N-substituierten 3-Cyano-azetidins nach Anspruch 8 zur entsprechenden N-substituierten Azetidin-3-carbonsäure und gewünschtenfalls ein Hydrieren des N-substituierten Derivats zur Azetidin-3-carbonsäure.

**Revendications**

1. Procédé de préparation de dérivés de 3-cyano-azétidine N-substitués de formule I:

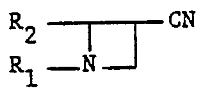

I

où $R_1$ représente un groupe benzyle et $R_2$ représente un atome d'hydrogène ou un groupe alcoyle, aryle ou aralcoyle dans lequel la fraction alcoyle contient jusqu'à 8 atomes de carbone, par réaction d'un dérivé d'azitidine-3-ol N-substitué de formule II:

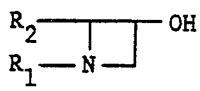

II

dans un solvant organique non-miscible à l'eau avec un halogénure d'alcoylesulfonyle, suivie par la réaction de la solution organique résultante du produit de 3-alcoylesulfonyl-azétidine avec une solution aqueuse d'un cyanure de métal alcalin en présence de, comme catalyseur de transfer t de phase lipophile, une proportion catalytique d'un halogénure de tétraalcoylammonium dans lequel les groupes alcoyles contiennent un total d'au moins 20 atomes de carbone.

2. Procédé selon la revendication 1 où $R_2$ représente un atome d'hydrogène.

3. Procédé selon la revendication 1 ou 2 dans lequel l'halogénure d'alcoylesulfonyle est le chlorure de méthylsulfonyle.

4. Procédé selon les revendications 1, 2 ou 3 dans lequel le solvant organique est le toluène ou le chlorobenzène.

5. Procédé selon l'une quelconque des revendications précédentes dans lequel l'halogénure de tétraalcoyleammonium contient au moins 2 groupes alcoyle contenant au moins 8 atomes de carbone et au moins un groupe méthyle.

6. Procédé selon l'une quelconque des revendications précédentes dans lequel le catalyseur de transfert de phase est présent en une quantité de 1 à 20% molaire, sur la base de l'alcool de départ.

7. Procédé selon l'une quelconque des revendications précédentes dans lequel la réaction entre l'alcool de formule II et l'halogénure d'alcoylesulfonyle est conduite en refroidissant pour maintenir la température de la réaction en dessous de 25°C et la réaction ultérieure avec un cyanure de métal alcalin conduite à température élevée.

8. Dérivés de 3-cyano-azétidine N-substitués de formule I lorsqu'on les prépare par un procédé selon l'une quelconque des revendications 1—7.

9. Procédé de préparation d'acides azétidine-3-carboxyliques N-substitués et d'acide azétidine-3-carboxylique dans lequel on hydrolyse une 3-cyano-azétidine N-substituée de la revendication 8 pour donner l'acide azétidine-3-carboxylique N-substitué correspondant et, si on le désire on hydrogène le dérivé N-substitué pour donner l'acide azétidine-3-carboxylique.